# EUROPEAN PATENT APPLICATION

(11) **EP 2 123 750 A1**
(43) Date of publication of application: **25.11.2009**
(21) Application number: 07860625.8
(22) Date of filing: 28.12.2007
(51) Int. Cl.: C12N 15/00, A01H 1/00, C12N 5/10, C12N 9/10, C12N 15/09

(54) **NOVEL PROTEIN HAVING ACYLTRANSFERASE ACTIVITY AND GENE ENCODING THE SAME**

(30) Priority: 28.12.2006 JP 2006356451
(71) Applicant: Kirin Holdings Kabushiki Kaisha, Tokyo 104-8288 (JP)
(72) Inventor: UMEMOTO, Naoyuki, Sakura-shi, Tochigi 329-1414 (JP); MOMOSE, Masaki, Sakura-shi, Tochigi 329-1414 (JP)
(74) Representative: HOFFMANN EITLE
(86) International application number: PCT/JP2007/075430
(87) International publication number: WO 2008/082006

(57) **Abstract**

This invention provides a means for obtaining flowering plants with a novel flower color, and a method for producing a plant having petals with a novel flower color by use of a novel acyltransferase protein and the gene encoding the same.

SEQUENCE LISTING

<110>  KIRIN HOLDINGS KABUSHIKI KAISHA

<120>  Novel protein having acyltransferase activity and gene encoding the same

<130>  PH-3420-PCT

<150>  JP 2006-356451
<150>  2006-12-28

<160>  15

<170>  PatentIn version 3.4

<210>  1
<211>  477
<212>  PRT
<213>  Dianthus caryophyllus

<400>  1

Met Thr Leu Ser Pro Leu Asp Asn Leu Val Leu Leu Ser Leu Leu Leu
1               5                   10                  15

Thr Pro Leu Leu Val Thr Gln Ala His Pro Arg Ile Ile Glu Ser Leu
            20                  25                  30

Pro Gly Tyr Lys Gly Gln Leu Pro Phe Thr Phe Glu Thr Gly Tyr Val
        35                  40                  45

Gly Val Gly Glu Arg Glu Glu Val Gln Leu Phe Tyr Tyr Phe Ala Glu
    50                  55                  60

Ser Glu Lys His Val Glu Ser Asn Pro Leu Met Leu Trp Leu Thr Gly
65                  70                  75                  80

Gly Pro Gly Cys Ser Ala Phe Ser Gly Leu Ala Phe Glu Ile Gly Pro
                85                  90                  95

Ile Ser Phe Asp Tyr Ala Arg Ser Asn Pro Glu Ser Asp Leu Pro Lys
            100                 105                 110

Leu Gln Leu Asn Pro Tyr Ser Trp Thr Lys Ser Ala Ser Ile Ile Phe
        115                 120                 125

Leu Asp Ser Pro Val Gly Ser Gly Tyr Ser Tyr Ala Arg Asn Tyr Glu
    130                 135                 140

Gly Tyr His Ser Asn Asp Thr Leu Gln Ser Ala His Ile Tyr Gln Phe
145                 150                 155                 160

Leu Thr Lys Trp Phe Leu Asp His Pro Asn Phe Lys Ser Asn Pro Leu
                165                 170                 175

Tyr Ile Ala Gly Asp Ser Tyr Ala Gly Met Ile Val Pro Arg Ile Val
            180                 185                 190

Gln Lys Ile Leu Asn Gly Asn Asp Asn Gly Leu Val Pro Lys Met Asn
        195                 200                 205

Leu Lys Gly Tyr Val Leu Gly Asn Pro Thr Thr Thr Gly Asn Lys Val
    210                 215                 220

Lys Tyr Ser Gln Ala Glu Tyr Glu Arg Arg Val Ser Leu Ile Pro Glu
225                 230                 235                 240

Glu Leu Tyr Gln Ser Ala Lys Ile Ser Cys Asn Gly Asp Phe Ser Asn
                245                 250                 255

Glu Asp Pro Arg Asn Val Lys Cys Ser Arg Ala Leu Glu Ala Ile Ser
            260                 265                 270

Glu Val Leu Ser Leu Val Asn Val Pro Tyr Ile Leu Glu Pro Ser Cys
        275                 280                 285

Val Pro Ile Ser Ser Lys Gln Thr Lys Lys Val Arg Arg Gln Met His
    290                 295                 300

Gln Met Glu Asp Asp His Ile Asn Ser Ile His Leu Arg Glu Gln Gln
305                 310                 315                 320

Gly Asp His Pro Trp Cys Arg Glu Asp Asn Tyr Ile Leu Ser Thr Ile
                325                 330                 335

Trp Thr Asn Asn Val Gln Val Gln Glu Ala Leu Asn Val Arg Lys Gly
            340                 345                 350

Thr Ile Asp Arg Trp Thr Arg Cys Asn Met Ser Ile Ser Tyr Ile Arg
        355                 360                 365

Asp Tyr Glu Ala Thr Val Val Gly Tyr His Lys Asn Phe Thr Asn Thr
    370                 375                 380

Asp Leu Gln Val Leu Leu Tyr Ser Gly Asp Gln Asp Leu Ala Ile Pro
385                 390                 395                 400

Tyr Val Gly Thr Ile Glu Trp Ile Lys Glu Leu Gln Ile Pro Thr Thr
                405                 410                 415

Asn Glu Trp Arg Pro Trp Phe Val Asp Gly Gln Val Ala Gly Tyr Val
            420                 425                 430

Thr Glu Phe Gly Gly Phe Ser Asp Lys Tyr Arg Leu Thr Tyr Thr Thr
        435                 440                 445

Val Lys Gly Ala Gly His Thr Ala Pro Glu Tyr Lys Pro Lys Glu Cys
    450                 455                 460

Phe Ala Met Ile Lys Arg Trp Leu Gly Leu Ser Ser Phe
465                 470                 475

<210>  2
<211>  1434
<212>  DNA
<213>  Dianthus caryophyllus

<400>  2
atgacacttt cgccgcttga taatttggtg cttttgtctc ttttattaac accgttactg      60

gttacacaag ctcacccacg tattatcgag tctctgcctg gttataaagg ccagcttccc     120

ttcacattcg aaactggata tgtaggtgtt ggagaaagag aggaagtgca gttattctat     180

tactttgcgg aatcagaaaa acatgttgaa agtaatccgc ttatgctttg gctcactggt     240

ggccctggtt gctctgcttt ttctggttta gcctttgaaa ttggtccaat atcatttgat     300

tatgcgagaa gcaatccaga atcagactta ccaaagcttc agttaaatcc atattcatgg     360

acaaagtcgg ctagcattat ttttctcgat tcaccggtgg ggtcaggcta ctcgtacgcc     420

aggaactacg agggatatca cagtaatgac actttgcagt ccgcccacat ttaccagttt     480

ttgactaagt ggtttctgga ccatccaaat ttcaaaagca atcctttgta cattgctggc     540

gattcatatg cgggaatgat tgtccccagg attgtgcaaa agatcttaaa tgggaatgac     600

aatggtcttg taccaaaaat gaatctgaag ggttatgttc tcggcaatcc aacgaccaca 660

ggaaataaag tcaaatattc acaagctgaa tatgaacgtc gcgtatcact catacctgag 720

gaactctacc agtcggccaa gatcagttgc aatggtgatt tttcaaatga ggatccaaga 780

aatgtaaagt gctcacgagc gcttgaagcc atatctgaag ttctaagttt ggttaacgta 840

ccatatatcc tggagccaag ttgtgtaccg atctcttcaa agcaaactaa gaaagttcga 900

agacagatgc atcagatgga agatgatcac atcaattcta ttcatttgcg tgagcagcaa 960

ggtgaccatc catggtgtcg agaagataat tacattcttt caaccatctg gactaacaat 1020

gtccaagttc aagaagcact caacgtccga aagggaacaa tagatcgttg gacgagatgt 1080

aatatgagca tatcgtacat cagggattat gaagccactg ttgtagggta ccataaaaat 1140

ttcacaaata cagatctcca agtactgctg tacagtggtg atcaagacct cgccatacct 1200

tatgttggaa caatagaatg gatcaaagag ctacaaatcc caaccacaaa cgaatggaga 1260

ccatggtttg ttgatggtca agttgccgga tacgtcacag agtttggagg cttctcggat 1320

aaataccggt taacctatac tactgttaag ggagctggac atactgctcc tgagtataag 1380

cccaaagagt gttttgcaat gatcaaaagg tggcttggat tatcctcttt ctag 1434

<210> 3
<211> 3646
<212> DNA
<213> Dianthus caryophyllus

<400> 3
cactacaaga aaatcttgta caggcgaccg aaaaaggcta ccaacaacag tcgctaaaac 60

aagcgggcga ctgactgtag tcgccagaca gcggtagcga ggctcagtcg ccattttttg 120

aggtcgggcg accgaaatca gtcgccaaat attggcgacc gacatatcag tcgccaaaaa 180

gggggcgggc gaccgacatc agtcgcccca tgtttcccct tttttaaaaa aaaaatgggg 240

gttggcgacc gacttcggtc gccatttatg aggcgggcga ccgcaagtcg gtcgccaggc 300

agtcgccatt tctctgtttt ttctaaattt ttgtaggatt cggcgaccga taatcagtcg 360

ccaccttaat atttcagtcg ccaaactact gcaaatttac aattgctgca cggtttcacg 420

ttttgcccgt attaatttca ttaccaaatt cagcaaacct gcaaattcaa cggtataacc 480

aacccataca atttcaacaa acagcataaa caaccattca aattcaacat tttcaaacag 540

aaattaagtt tcatacatgc aagtttagta tgcaacacat aattcaagta aacatgttca 600

acatccgcta acagtttgca taaacggaaa aagagatact ttacacttga aacccaacag 660

atgctccact actcgggcca ccaccatgag ggtctctagg gtcaggaaac tgcggaccaa 720

tccctggatt acactgctca aagtaggtct gcatccgctg caattgttcc ctcatcaacc 780

tgagctcctc gtcttgagca gaaaggcgtt cgtcttgatt ggcaacgcga tcttctaatt 840

tccttacaat acccggcgtg tacatggagt cgctcactgt cgacaatctg cggtttctct 900

tctcgtaaat atactcggct gcgttgccga ctccataaac cgcccctttt ttaaaccctt 960

cgttcacttc caaccaaatt tgatcgggtt tagttccagg ctcctcctcg actcgcttca 1020

tatacgcctc ctaccaaaga gcaaatcaat tatgctattt aattacaaca aattaaaaag 1080

attataaatc tcattacgac caacaattcc ttacatattt atccgagtct ttctttctcg 1140

cgaaatgcaa acggccttcc tcgtcttttt tggcgtgcgt gtccaaaaaa tgtcaaacgc 1200

ggtcgccgct tgtcctttgt ttgcctttag tccaaaaaac ggcaacaatt atccatttaa 1260

atatacacaa tcatactata tattattgga ttatgtaaac gatatattaa aacttacttt 1320

ttctagttct tgtgcacgat caaggcagct ctgagatcct ctaaagtaac tcggctcgac 1380

gcgcgcattt ggatctcctc ccctaatatt caacgccgct tgtttggatt tcctttgaaa 1440

ttccggtgtg tctcgtaact taagcaagtc gttgaatgca acatctacaa gaaaaaatat 1500

gacgacaacc aaataaaacc gaacaacaaa ttgccgaaat atcaatttta taacctcatt 1560

tttattaaag ttagttttat taccattcat gaatgcgggc ttcttctttc ttttgacagc 1620

cttccacatg ttgtccttga agcgtttttg acacgttttc tcgtactttg cacggactaa 1680

atgctcaaga accggattcc aagtgcacga attctacgta taaaaccaaa tattaaacaa 1740

attccggcgt aataatatat atacatttta aaaacactag tggctttaaa aattatatac 1800

gtacccggaa aatattaaac cactgctcct tcaagtcgtc tggtatcgac gaccaacaag 1860

tgtaaccgga agtgtaactg ctttttgtac tctccgtaca tgccttagta attttctttt 1920

gcggaaacct tacaattttt gcgttaaaat aataagcaat atgaacaact taccaatatg 1980

attaacttac caatataaac aacttaccaa tatgatttcg ggtctaaaat taatttgccg 2040

gtcgcatctg ttgggggggt gtcgtccgcc tcctgctctt cgttttgttg ttgcgtctcc 2100

tgactatccg acgactcagt ctcctcgtcg gacctcattg acgatctgga ggaactacct 2160

tttctgcttc gccctgacga cataattcaa actaaaaaaa aaacaacatt aacggggcct 2220

attattaaat gagaacaagt tagtaacaca ctactaactt gtcaaatctt tcctcataca 2280

tcccattcgc gctccaatct cttcataata ggtcgttttc tcttatcaaa tctttcctca 2340

tacatccatt cgcgctccaa tctcttcatt attataagct aaccaatata agagaaataa 2400

ttcaataccc aaaagttcaa acaagtaatt taaccaactt aatatttgag aactaatttc 2460

aacttaatat ttgagaactt aacaaaaaaa cctcaaactc acaatcccta aattttattg 2520

aaatcaatcc tacattaata gaaaataata acactaatct taatcaaaac atattctaac 2580

ataaaaataa taatctaaca ttatctaatc ttacataaca tttaaacatt ctctaatctt 2640

aaactatcat tcaaacattc tctaatctta aataaattct ctaatataca cattggtgta 2700

tgaggctaaa tccaaccaac aacatcaaag aatagaagct tatttaggct ccaccaacca 2760

acatccaacc aaccaacaac atcaaattct ctaatataca aatgctcaaa tcaaaaattc 2820

tctcatgtaa gctccaccaa caacatcaaa ccacactatt aaggccaatt caaagcttat 2880

ttaggctatt atatcaaact taactcaaat tagaaacttc aagaatacac aaatgtcaaa 2940

accgctcctc tacgaatccg tgctctacaa ataaaccatt ctactatcaa ataccatctt 3000

aaaactacct aattaacaca ctaattcaat aaaatcggct ataaatcgaa aacccctaat 3060

ttctagggtt agggtttgac gaaaattaaa ttaaaacgat taactacgaa tcataccttt 3120

aacaactcga ttaactaggc tagaacttca aatcccgaca aaaacaacca aaagtcgacc 3180

caaaaacggc cccaacgaaa acagcggaga agatggtggt gcgtgaatgg tggtgatgcg 3240

gtggcggtgg tgcggtgacg gcggcacggt gcggtgttgg tggtgcgtga atggtggtga 3300

tggtggtgga tgatgaatga agatggtggt tgatgaagac ggtggttgca ctactcttaa 3360

tctccttttt tttttttttt tttctaagta taatcgaaag agtaagttag ggggagaagc 3420

tgcggatgaa tctaaaatgt ggggcgactg aattggcgac cgaagtcggt cgcccgatat 3480

ttttttggcg accgaagtcg gtcgcccact tatttttttt tagtcgcccc ttagtcgcct 3540

ttggtcgact aatttcagtc gcccgatcaa tatttcggtc gcctgttagc gactacatta 3600

ttcagtcgcc attttacggt cgcccgtaat agattttctt gtagtg 3646

<210> 4
<211> 29
<212> DNA
<213> Artificial

<220>
<223> Primer

<220>

<221>  misc_feature
<222>  (18)..(18)
<223>  n is a, c, g, or t

<220>
<221>  misc_feature
<222>  (21)..(21)
<223>  n is a, c, g, or t

<220>
<221>  misc_feature
<222>  (24)..(24)
<223>  n is a, c, g, or t

<220>
<221>  misc_feature
<222>  (27)..(27)
<223>  n is a, c, g, or t

<400>  4
atgatttggc ttacaggngg nccnggntg                                          29

<210>  5
<211>  20
<212>  DNA
<213>  Artificial

<220>
<223>  Primer

<220>
<221>  misc_feature
<222>  (9)..(9)
<223>  n is a, c, g, or t

<220>
<221>  misc_feature
<222>  (12)..(12)
<223>  n is a, c, g, or t

<220>
<221> misc_feature
<222> (15)..(15)
<223> n is a, c, g, or t

<400> 5
gctgtrtgnc cngcnccytt 20

<210> 6
<211> 24
<212> DNA
<213> Artificial

<220>
<223> Primer

<400> 6
ggttgctctg ctttctctgg cctc 24

<210> 7
<211> 24
<212> DNA
<213> Artificial

<220>
<223> Primer

<400> 7
ttaactgtag cataaactaa gcgg 24

<210> 8
<211> 25
<212> DNA
<213> Artificial

<220>
<223> Primer

<400> 8

gggtgctctt cttggaacgg tctcg 25

<210> 9
<211> 25
<212> DNA
<213> Artificial

<220>
<223> Primer

<400> 9
cccttgactg tcgcgtacgt caaag 25

<210> 10
<211> 20
<212> DNA
<213> Artificial

<220>
<223> Primer

<400> 10
ctgctttttc tggtttagcc 20

<210> 11
<211> 20
<212> DNA
<213> Artificial

<220>
<223> Primer

<400> 11
cagtagtata ggttaaccgg 20

<210> 12
<211> 20
<212> DNA

<213>  Artificial

<220>
<223>  Primer

<400>  12
gctctgcttt gtcgggcctc                                                    20


<210>  13
<211>  20
<212>  DNA
<213>  Artificial

<220>
<223>  Primer

<400>  13
accgtagcat agactaatcg                                                    20


<210>  14
<211>  22
<212>  DNA
<213>  Artificial

<220>
<223>  Primer

<400>  14
gaatatgaac gtcgcgtatc ac                                                 22


<210>  15
<211>  21
<212>  DNA
<213>  Artificial

<220>
<223>  Primer

<400>  15

cattgcaact gatcttggcc g 21

## Description

### Technical Field

The present invention relates to a novel protein having acyltransferase activity, a gene encoding the same, and a method for producing a plant having the gene. Specifically, the gene encoding the novel protein having acyltransferase activity of the present invention is introduced into a plant lacking such gene or a plant not having acyltransferase activity, such as a plant lacking a novel acyltransferase or a plant in which a novel acyltransferase does not function (e.g., a plant other than a plant belonging to the genus Dianthus (e.g., a carnation)). Thus, breeding of an unprecedented plant containing acylated anthocyanins comprising malic acids (i.e., a plant having a novel flower color) can be realized.

### Background Art

The most important property of flowering plants is a flower color. Among the flower colors, anthocyanins are deeply involved in pink, red, blue, and purple flower colors and are important. To obtain various flower colors, breeding has been performed so far by crossing, mutations, or genetic recombination. Particularly, bicolor varieties, which have two flower colors in one petal, have highly been prized. However, genes or genetic backgrounds thereof have not been elucidated at the genetic level.

Anthocyanins have been known to become molecules having various color tones as a result of modifications with various acyl groups. As their molecular mechanisms have been elucidated in recent years, the presence of plant BAHD acyltransferases having common sequences and a group of genes encoding them has been revealed (see Non-Patent Document 1).

Pigments in the petals of carnations, one of three major horticultural flowering plants, are usually anthocyanins having acyl groups (see Non-Patent Document 2). This acyl group in carnations is characterized by being modified with malic acid, unlike those in other plants. Varieties free of acyl groups (e.g., 'Nazareno', Kirin Agribio Co., Ltd.) have been bred in recent years, and a group of carnations having obviously different flower colors from conventional ones have been produced (see Non-Patent Documents 3 and 4). However, molecular mechanisms to produce such unique flower colors of carnations have not been elucidated yet.
Non-Patent Document 1: D'Auria, Curr. Opin. Plant Biol., 9, 331-340 (2006)
Non-Patent Document 2: M. Nakayama et al., Phytochemistry, 55, 937-939 (2000)
Non-Patent Document 3: M. Yamaguchi, Report of Research of Faculty of Horticulture, Minamikyushu University, 19, 1-78 (1989)
Non-Patent Document 4: H. Yoshida et al., Journal of Horticulture ("Engeigaku Zasshi"), 72, suppl. 1, 130 (2003)

### Disclosure of the Invention

It is an object of the present invention to provide a flowering plant having a novel flower color by using a gene capable of transferring malic acids as acyl groups to anthocyanins and causing expression of the gene in petals.

The present inventors have conducted diligent studies on molecular mechanisms to produce the unique flower colors of carnations. As a result, the present inventors have newly found a gene encoding a novel protein having acyltransferase activity from carnations and have confirmed that this gene functions in carnations. Specifically, it has been revealed that a gene encoding a protein having the activity of transferring acyl groups in carnations does not belong to a group of plant BAHD acyltransferase genes known in the art. From bicolor breeding lines having both of non-acylated anthocyanins free of acyl groups and acylated anthocyanins having acyl groups, it has been confirmed that the gene is interrupted by insertion of a transposon, and that the transposon has been excised in petals comprising acylated anthocyanins. The present inventors have further shown that the mutant gene interrupted by this transposon displays simple inheritance. The present inventors have completed the present invention by finding that breeding of a novel plant having acylated anthocyanins containing malic acids (i.e., a flower having a novel flower color) can be realized by introducing a gene encoding a novel protein having acyltransferase activity into a plant lacking such gene or a plant not having acyltransferase activity, such as a plant lacking a novel acyltransferase or a plant in which a novel acyltransferase does not function (e.g., a plant other than a plant belonging to the genus Dianthus (e.g., a carnation)).

Specifically, the present invention encompasses the following inventions.
[1] A protein which is the following (a) or (b):
   (a) a protein consisting of the amino acid sequence set forth in SEQ ID NO: 1; or
   (b) a protein consisting of an amino acid sequence derived from the amino acid sequence set forth in SEQ ID NO: 1 by deletion, substitution, insertion, or addition of 1 or more amino acid(s) and having the activity of transferring malic acids as acyl groups to anthocyanins.
[2] A gene consisting of DNA which is one of the following (c) to (f):
   (c) DNA consisting of the nucleotide sequence set forth in SEQ ID NO: 2;
   (d) DNA which hybridizes under stringent conditions to DNA consisting of a nucleotide sequence complementary to the nucleotide sequence set forth in SEQ ID NO: 2 and encodes a protein having the activity of transferring malic acids as acyl groups to anthocyanins;
   (e) DNA which consists of a nucleotide sequence having a homology of 80% or higher to the nucleotide sequence set forth in SEQ ID NO: 2 and encodes a protein having the activity of transferring malic acids as acyl groups to anthocyanins; or
   (f) DNA consisting of a degenerate isomer of the nucleotide sequence set forth in SEQ ID NO: 2.
[3] A recombinant vector containing the gene according to [2].
[4] A plant cell to which the recombinant vector according to [3] has been introduced.
[5] A method for producing a gene recombinant plant having petals with a novel flower color, comprising introducing the gene according to [2] into a plant cell so as to regenerate a plant from the plant cell.
[6] The method for producing a gene recombinant plant having petals with a novel flower color according to [5], wherein a plant into which the gene is introduced is a plant other than a plant belonging to the genus *Dianthus*.

This description includes part or all of the contents as disclosed in the description and/or drawings of Japanese Patent Application No. 2006-356451, which is a priority document of the present application.

### Brief Description of the Drawings

FIG. 1 is a scheme showing a reaction catalyzed by a novel acyltransferase. FIG. 1A shows a reaction through which malic acids are added to anthocyanins having glucose at the position 3, and FIG. 1B shows a reaction through which anthocyanins having glucose at the positions 3 and 5 are converted to cyclic malyl anthocyanins.
FIG. 2 is a photograph of a flower of an MFA1 line.

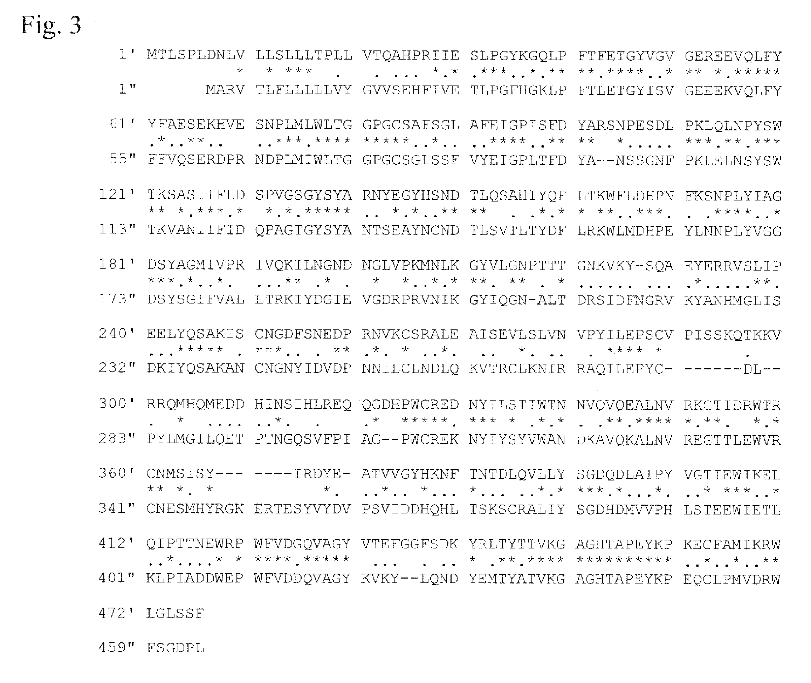

FIG. 3 is a figure showing the results of analyzing, with DNA analysis software GENETYX (ver. 4.0, Genetyx Corp.), the amino acid sequences of a protein (upper row) predicted from a cDNA sequence encoding a noble protein having acyltransferase activity and a glucose/isobutyric acid transfer protein (lower row) known in the art, which synthesizes tomato acyl acetals (substances resistant to insects) and was used to isolate the gene of the present invention. These amino acid sequences exhibit 44.6% homology as a whole, though partial more homology is observed. In the figure, the marks "*" and "." denote identical amino acids and homologous amino acids, respectively.
FIG. 4 is a figure showing an intron sequence in which a transposon has been inserted. In the figure, TSD is underlined.
FIG. 5 is a figure showing a footprint confirmed in the variegated spot of a bicolor line after the excision of the transposon. Two footprints could be confirmed for an MFA1-3 line.

### Best Mode for Carrying Out the Invention

The present invention is hereafter described in greater detail.

### 1. Novel acyltransferase

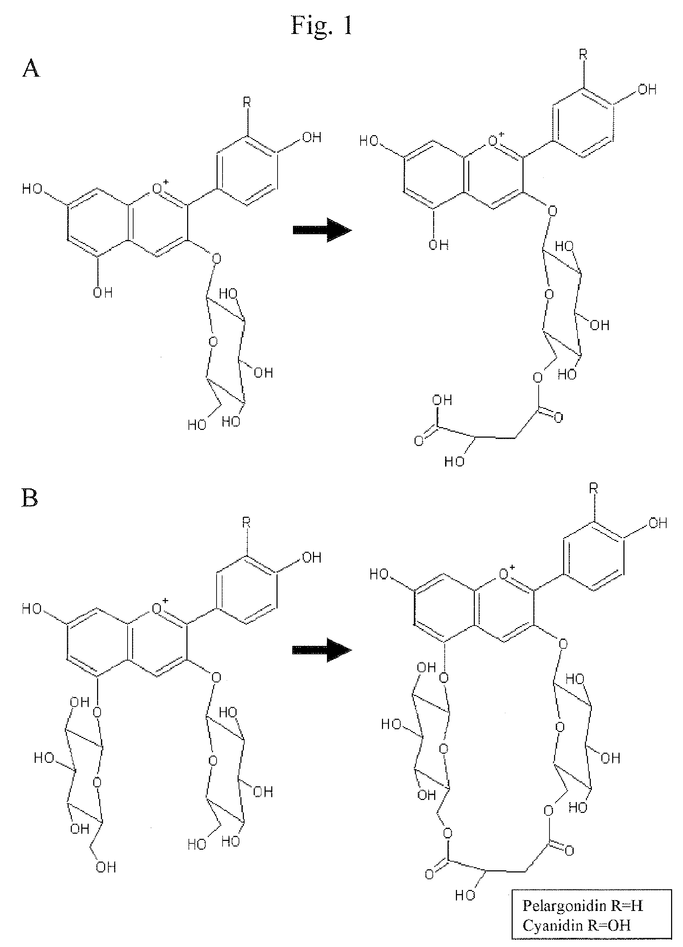

The protein of the present invention is a novel acyltransferase having the activity of adding malic acids as acyl groups to anthocyanins having glucose at the position 3 and the activity of converting anthocyanins having glucose at the positions 3 and 5 into cyclic malyl anthocyanins. FIG. 1A shows a reaction through which malic acids are added to anthocyanins having glucose at the position 3, and FIG. 1B shows a reaction through which anthocyanins having glucose at the positions 3 and 5 are converted to cyclic malyl anthocyanins.

The amino acid sequence of the protein of the present invention is set forth in SEQ ID NO: 1.

### 2. The gene encoding a novel protein having acyltransferase activity

The gene of the present invention is a gene encoding a novel acyltransferase having the activity of adding malic acids as acyl groups to anthocyanins having glucose at the position 3 and the activity of converting anthocyanins having glucose at the positions 3 and 5 into cyclic malyl anthocyanins.

The nucleotide sequence of the gene of the present invention is set forth in SEQ ID NO: 2.

The protein of the present invention includes a novel protein consisting of an amino acid sequence derived from the amino acid sequence set forth in SEQ ID NO: 1 by deletion, substitution, insertion, or addition of one or more amino acids and having the above novel acyltransferase activity. The range of the phrase "one to several" is not particularly limited and means, for example, approximately 1 to 20, preferably, approximately 1 to 10, more preferably, approximately 1 to 7, even more preferably, approximately 1 to 5, particularly preferably, approximately 1 to 3.

The deletion, substitution, insertion, or addition of amino acid(s) can be performed by modifying a gene encoding the protein by an approach known in the art. The modification can be introduced into the gene by an approach known in the art such as a Kunkel or Gapped duplex method or by a method equivalent thereto. For example, the modification is introduced thereinto by use of a modification introduction kit using a site-specific mutagenesis method (e.g., Mutant-K (manufactured by TAKARA BIO Inc.) or Mutant-G (manufactured by TAKARA BIO Inc.)) or by use of an LA PCR *in vitro* Mutagenesis series kit manufactured by TAKARA BIO Inc.

The term "novel acyltransferase activity" refers to the activity of adding malic acids as acyl groups to anthocyanins having glucose at the position 3 and the activity capable of converting anthocyanins having glucose at the positions 3 and 5 to cyclic malyl anthocyanins. When a certain protein has "an activity substantially equivalent to the activity of the protein consisting of the amino acid sequence set forth in SEQ ID NO: 1," it can be said that the protein has the novel acyltransferase protein activity. In this context, the phrase "activity substantially equivalent" means that the specific activity of the enzyme is determined to be an activity with 80% or higher, preferably 90% or higher identity to the activity of the protein consisting of the amino acid sequence set forth in SEQ ID NO: 1.

Whether or not such a mutant protein actually has the novel acyltransferase protein activity can be confirmed by: preparing a vector comprising a promoter (e.g., a cauliflower mosaic virus 35S RNA promoter) ligated upstream of the gene encoding the protein; introducing the vector into a plant accumulating therein non-acylated anthocyanins (e.g., Nazareno (Kirin Agribio Co., Ltd.)) by various transformation methods (described later) that are conventionally well known and commonly used; and then measuring pigments in petals.

The gene encoding the protein having the amino acid sequence set forth in SEQ ID NO: 1 of the present invention can be obtained by preparing a primer based on the amino acid sequence set forth in SEQ ID NO: 1, extracting mRNA from carnation petals, and carrying out a reverse transcription polymerase chain reaction (sometimes referred to as RT-PCR) by use of the primer and the mRNA serving as a template, followed by amplification.

Specifically, the gene encoding the novel protein having acyltransferase activity of the present invention is DNA consisting of the nucleotide sequence set forth in SEQ ID NO: 2.

In addition, the gene encoding the novel protein having acyltransferase activity of the present invention includes DNA that hybridizes under stringent conditions to DNA consisting of a nucleotide sequence complementary to the nucleotide sequence set forth in SEQ ID NO: 2 and encodes a novel protein having acyltransferase activity.

In this context, the stringent conditions refer to conditions under which so-called specific hybrids are formed while non-specific hybrids are not formed, and refer to, for example, conditions involving a sodium concentration of 10 mM to 300 mM, preferably 20 to 100 mM, and a temperature of 25°C to 70°C, preferably 42°C to 55°C.

Further, the gene encoding the novel protein having acyltransferase activity of the present invention includes DNA that consists of a nucleotide sequence at least 80%, preferably at least 90%, and the most preferably at least 95% homologous to the nucleotide sequence set forth in SEQ ID NO: 2 and encodes the above novel protein having acyltransferase activity. In this context, the homology percentage is calculated from default (initial setting) parameters by use of a nucleotide sequence comparison program (e.g., DNASIS-Mac v. 3.7 (Hitachi Software Engineering Co., Ltd.) or GENETYX ver. 4.0 (Genetyx Corp.)). The gene encoding the novel protein having acyltransferase activity of the present invention further encompasses a degenerate isomer thereof. In this context, the degenerate isomer means DNA that is different therefrom in a degenerate codon and can encode the same protein. For example, DNA having the nucleotide sequence of SEQ ID NO: 2 but having the substitution of a codon corresponding to a certain amino acid by a codon having a degenerate relationship therewith is referred to as a degenerate isomer in the present invention. For example, DNA having the nucleotide sequence of SEQ ID NO: 2 but having the substitution of a codon (AAC) corresponding to Asn by a codon having a degenerate relationship therewith, for example, AAT, is referred to as a degenerate isomer in the present invention.

The above mutant gene (mutant DNA) can be prepared by an approach known in the art such as a Kunkel or Gapped duplex method or by a method equivalent thereto, for example, by use of a mutation introduction kit using a site-specific mutagenesis method (e.g., Mutant-K (manufactured by TAKARA BIO Inc.) or Mutant-G (manufactured by TAKARA BIO Inc.)) or by use of an LA PCR *in vitro* Mutagenesis series kit manufactured by TAKARA BIO Inc. The mutagenesis method can be selected and practiced by those skilled in the art without particular difficulties with reference to the nucleotide sequence of the gene according to the descriptions of a document such as Molecular Cloning [Sambrook et al. ed., 15, Site-directed Mutagenesis of Cloned DNA, 15.3 to 15.113, Cold Spring Harbor Lab. Press, New York (1989)]. Alternatively, techniques for artificially performing the substitution, deletion, insertion, or addition of one or more (1 or several or more) bases from the nucleotide sequence (site-specific mutagenesis) can be practiced by those skilled in the art on the basis of the nucleotide sequence of the gene according to techniques described in, for example, Proc. Natl. Acad. Sci. USA, 81, 5662-5666 (1984); WO85/00817; Nature, 316, 601-605 (1985); Gene, 34, 315-323 (1985); Nucleic Acids Res., 13, 4431-4442 (1985); Proc. Natl. Acad. Sci. USA, 79, 6409-6413 (1982); and Science, 224, 1431-1433 (1984).

### 3. Recombinant vectors

The recombinant vector of the present invention can be constructed by introducing the gene described in 2. above into an adequate vector. Herein, a preferred example of such vector that can be used is a vector derived from pBI, pPZP, or pSMA, with which a target gene can be introduced into a plant by use of the *Agrobacterium*. In particular, a binary vector derived from pBI or an intermediate vector system is preferably used. Examples of such vector include pBI121, pBI101, pBI101.2, and pBI101.3. A binary vector is a shuttle vector that can be replicated in colon bacillus (*Escherichia coli*) and *Agrobacterium.* When a plant is caused to be infected with *Agrobacterium* carrying a binary vector, a partial DNA that is sandwiched by border sequences (an LB sequence and an RB sequence) located on a vector can be incorporated into a nuclear DNA of a plant [EMBO Journal, 10(3), 697-704 (1991)]. Meanwhile, in the case of a vector derived from pUC, a gene can be introduced directly into a plant. Examples of such vector include pUC18, pUC19, and pUC9. In addition, plant virus vectors such as a cauliflower mosaic virus (CaMV), a bean golden mosaic virus (BGMV), and a tobacco mosaic virus (TMV) can be used.

When a gene is inserted into a vector, a method wherein isolated DNA, which is used for insertion, is cleaved with an adequate restriction enzyme, the obtained DNA fragment is inserted into a restriction enzyme site or a multicloning site of an adequate vector DNA, and the gene is ligated to the vector DNA is used, for example.

It is necessary for the above gene to be incorporated into a vector in a manner such that functions of the gene are exerted. Thus, a vector may comprise constituents such as a promoter, an intron, an enhancer, a translation termination codon, a terminator, a polyA additional signal, and a 5'-UTR sequence in the upstream or downstream region of or inside of the gene. In addition, such vector may comprise a selection marker gene. For such constituents, those known to the public may be used in combination according to need.

Examples of a promoter that may be used include a systemically-expressed promoter and a promoter that is known to function in a petal to result in a target gene (i.e., the gene encoding the novel protein of the present invention) being expressed in a petal. For instance, examples of a systemically-expressed promoter include a cauliflower mosaic virus 35S promoter (35SP). Examples of a promoter that is known to function in a petal include an EPSP synthase promoter and a chsA promoter of petunia. In addition, examples of a promoter that may be used include: promoters derived from a promoter of an isopentenyl transferase (ipt) gene or a nopaline synthase (nos) gene of the *Agrobacterium* and a promoter of a highly-expressed gene selected from a genome of a plant serving as a host used for transformation [Genschik et al., Gene, 148, 195-202 (1994)]. Further, a promoter exhibiting a very high level of promoter activity, which is a chimeric promoter obtained by combining a plurality of the above promoters, can also be used [Plant J., 7, 661-676 (1995)].

Note that promoters that can be used in the present invention are not limited to the above examples as long as the promoters are known to function in petals. Such promoters can be obtained by an amplification reaction of PCR by use of primers that are designed based on the nucleotide sequences of DNAs comprising the above promoters and genomic DNA as a template. In such case, an example of template DNA that can be used for PCR is genomic DNA of a cauliflower mosaic virus.

In addition, if necessary, it is possible to introduce an intron sequence having a function of enhancing gene expression, such as an intron of corn alcohol dehydrogenase (Adh1) [Genes & Development, 1, 1183-1200 (1987)], between a promoter sequence and a gene.

Examples of an enhancer that can be used include a virus-derived translation enhancer and a plant-derived translation enhancer. Examples of such virus-derived translation enhancer include sequences of tobacco mosaic virus, Alfalfa mosaic virus RNA4, bromo mosaic virus RNA3, potato virus X, and tobacco H virus [Gallie et al., Nuc. Acids Res., 15, 8693-8711 (1987)]. In addition, examples of such plant-derived translation enhancer include a sequence derived from soybean β-1, 3 glucanase (Glu) [written by Isao Ishida and Norihiko Misawa, edited by Kodansha Scientific, "Manuals for Cell Engineering Experimental Operations (Saibo-Kogaku Jikken Sousa Nyumon)," Kodansha Ltd., p. 119 (1992)] and a sequence derived from a tobacco ferredoxin-binding subunit (PsaDb) [Yamamoto et al., J. Biol. Chem., 270, 12466-12470 (1995)]. Examples of a translation termination codon include sequences of TAA, TAG, and TGA [e.g., Molecular Cloning described above].

As a terminator, a sequence capable of terminating transcription of a target gene transcribed by the aforementioned promoter may be used. Examples thereof include a terminator (nosT) af a nopaline synthase (nos) gene, a terminator of an octopine synthase (ocs) gene, and a terminator of a CaMV 35S RNA gene [Annu. Rev. Plant Physiol. Plant Mol. Biol., 44, 985-994 (1993); Plant Genetic Transformation and Gene Expression; a laboratory manual, edited by J. Draper et al., Blackwell Scientific Publication (1988)].

In addition, it has been reported that an enhancer region of a 35S gene was identified as a transcription enhancer inside of a promoter, and ligation of a plurality of such regions resulted in the improvement of the activity [Plant Cell, 1, 141-150 (1989)]. Such region also can be used as a part of a recombinant vector.

Examples of a selection marker gene include an ampicillin-resistant gene, a neomycin-resistant gene, a hygromycin-resistant gene, and a bialaphos-resistant gene. As described above, a recombinant vector may be prepared by ligating a selection marker gene to a plasmid to which a target gene is also ligated. Alternatively, a recombinant vector obtained by ligating such selection marker gene to a plasmid and a recombinant vector obtained by ligating a target gene to a plasmid may be separately prepared. In such case, the obtained vectors may be cotransfected into a host.

Preferably, these various types of constituents are incorporated into a recombinant vector in a manner such that each constituent can function in accordance with its properties. Operations for such incorporation can adequately be carried out by those skilled in the art.

### 4. Transformed plants

By use of the recombinant vector prepared in 3. above, it is possible to prepare a transformed plant by transforming cells of a plant of interest and regenerating such plant.

When a transformed plant is prepared, a variety of methods that have been reported and established can adequately be used. Preferred examples of such method for gene introduction include: a biological method using a Ti plasmid and an Ri plasmid of *Agrobacterium* and a virus as a vector; and a physical method using electroporation, polyethylene glycol, particle gun, or microinjection [Plant Genetic Transformation and Gene Expression; a laboratory manual, edited by J. Draper et al., Blackwell Scientific Publication (1988)] or silicon nitride whiskers [Euphytica, 85, 75-80 (1995); In Vitro Cell. Dev. Biol., 31, 101-104 (1995); and Plant Science, 132, 31-43 (1998)]. Such a method for gene introduction can adequately be selected and used by those skilled in the art.

In general, a gene introduced into a plant is incorporated into the genome of a host plant. Upon such incorporation, a phenomenon referred to as a "position effect" is observed, in which the expression of a transgene varies depending on the position on the genome into which the transgene is introduced. Thus, it is necessary to confirm that a transgene has been incorporated into a plant. Further, it is necessary to confirm the position of a transgene.

It is possible to confirm whether or not a gene has been incorporated into a plant by PCR, Southern hybridization, Northern hybridization, Western blotting, or the like. For instance, DNA is prepared from a transformed plant and primers specific to the DNA are designed, followed by PCR. After PCR, an amplified product is subjected to agarose gel electrophoresis, polyacrylamide gel electrophoresis, capillary electrophoresis, or the like, followed by staining with ethidium bromide, an SYBR Green solution, or the like. Then, the completion of transformation can be confirmed by detecting an amplified product expressed in the form of a single band. In addition, it is also possible to perform PCR using a primer which has previously been stained with a fluorescent pigment or the like so as to detect an amplified product. Moreover, a method wherein an amplified product is allowed to bind to a solid phase such as a microplate following which the amplified product is confirmed by a fluorescence or enzyme reaction or other means may be used.

Examples of a plant to be transformed using the gene encoding the novel acyltransferase of the present invention include: monocotyledons such as foliage plants of the families *Liliaceae, Orchidaceae*, and *Araceae*; and dicotyledons such as potatoes, chrysanthemums, roses, carnations, petunias, *Gypsophila muralis*, cyclamens, asters, salvias, and gentians. Particularly preferred examples of plant types include: chrysanthemums, carnations, and roses, which are three major flowering plants in terms of production, distribution, and consumption throughout the world; and petunias (clones), which have been increasingly produced, distributed, and consumed throughout the world in recent years.

Among the above examples, a plant to be transformed using the gene encoding the novel acyltransferase of the present invention is specifically a plant not having the activity of the novel acyltransferase of the present invention (i.e., a plant lacking the novel acyltransferase of the present invention or a plant in which the novel acyltransferase of the present invention does not function). Examples of such plant include a plant other than a plant belonging to the genus Dianthus (e.g., a carnation).

In the present invention, examples of plant materials to be transformed by use of the gene of the present invention in order to produce a transformed plant body include cells of growing points, shoot primordia, meristems, folia, stem pieces, root pieces, tuber pieces, sepal pieces, protoplasts, calluses, anthers, pollens, pollen tubes, peduncle pieces, flower stem pieces, petals, sepals, and the like.

In the cases of plant cells that are used for transformation, in order to regenerate a transformed plant from a transformed cell obtained, known tissue culture methods may be used. Those skilled in the art can readily carry out operations for such regeneration by use of methods of regenerating a plant from a plant cell that are known to the public. Regarding regeneration of a plant from a plant cell, it is possible to refer to references such as "Plant Cell Culture Manual (Shokubutsu Saibo Baiyo Manual)" [written and edited by Yasuyuki Yamada, Kodansha Scientific, 1984).

Specifically, a transformed plant cell is cultured in a medium used for callus formation, which is sterilized following the addition of mineral nutrients, vitamins, carbon sources, sugars serving as energy sources, plant growth regulators (phytohormones such as auxin and cytokinin), and the like. Then, formation of a dedifferentiated callus that grows in an indefinite manner is induced (such induction being hereafter referred to as "callus induction"). The thus formed callus is transferred to a new medium containing plant growth regulators such as auxin, followed by further proliferation (subculture).

For instance, callus induction may be carried out using a solid medium made of agar or the like and subculture may be carried out in a liquid culture. In each culture, mass cultivation can be efficiently carried out. Next, a callus proliferated via the aforementioned subculture is cultured under adequate conditions, resulting in induction of redifferentiation of organs (hereafter referred to as "induction of redifferentiation"). Eventually, a complete form of a plant is regenerated. The induction of redifferentiation can be carried out by adequately predetermining the types and amounts of constituents such as plant growth regulators (e.g., auxin and cytokinin) and carbon sources in a medium, lighting, temperatures, and the like. As a result of such induction of redifferentiation, adventitious embryos, adventitious roots, adventitious buds, adventitious stems and leaves, and the like are formed, leading to the growth of a further complete plant. Alternatively, such plant at a state before it has reached its complete form (in the form of a capsulated artificial seed, a dried embryo, a lyophilized cell or tissue, or the like) may be preserved such that a plant is regenerated by culture or the like according to need.

In addition, it is also possible to regenerate a transformant plant by culture/induction of proliferation of a transformed plant cell without callus induction by adequately predetermining conditions related to the types and amounts of different constituents, lighting, temperatures, and the like.

In a transformed plant into which the novel acyltransferase of the present invention has been introduced, the novel acyltransferase functions in petals in a manner such that malic acids as acyl groups are transferred to anthocyanins. Specifically, malic acids as acyl groups are added to anthocyanins having glucose at the position 3, and anthocyanins having glucose at the positions 3 and 5 are converted to cyclic malyl anthocyanins. As a result, acylanthocyans accumulate in petals. Thus, it has become possible to obtain plants having unprecedented petal colors. For instance, in a case in which the novel acyltransferase of the present invention is introduced into a petunia not containing such acyltransferase, flowers having petals containing anthocyanins comprising malic acids can be obtained. More specifically, a red petunia (e.g., the variety "baccarat red," Sakata Seed Co.) contains cyanidin-3-glucoside as a main pigment. Breeding of an unprecedented petunia containing, as a pigment, cyanidin-3-malyl glucoside can be realized by introducing the novel acyltransferase into the above petunia and causing expression therein.

The flower color can be determined by visual observation or can be determined by a colorimetric measurement test. In the colorimetric measurement test, the chromaticity of the petals of three individuals from each line is measured three times with a simple spectroscopic colorimeter (NF333 type) manufactured by Nippon Denshoku Industries Co., Ltd. in the fully opened state of the petals of plants that have bloomed. The average value thereof can be calculated to thereby evaluate flower colors. In this context, the colorimetric measurement is alternative means for describing the phenotypes of observed colors. It should be regarded as an index for recognized colors and does not limit obtainable potential colors.

The scope of the transformed plants of the present invention includes progeny plants and individuals obtained by any means of cultivation or breeding by use of the "T1 generation." Examples thereof include: plants of the "T1 generation" (redifferentiated generation as a result of transformation treatment); progeny plants of the "T2 generation" obtained using T1 seeds via self-pollination or cross-pollination; "T2-generation" plants that are found to be transgenic plants via drug screening, a Southern analysis method, or the like; progeny plants of the next generation after the T2 generation ("T3 generation") obtained via self-pollination or cross-pollination of plant flowers; individuals obtained via proliferation and maintenance of T1-generation plants by use of clones; and mutant individuals and the like that are progeny plants derived from the "T1 generation" and the like in which specific properties are changed.

Hereinafter, the present invention will be described specifically with reference to Examples. However, the present invention is not intended to be limited to these

### Examples.

Carnation lines or varieties used in Examples below have been stored in Kirin Agribio Co., Ltd. and can be purchased or requested for transfer as experimental materials. Contact information: Nichibei Bldg. 8F, 2-24-2 Hacchobori, Chuo-ku, Tokyo, 104-0032, Japan, Phone: 03-5541-5875, Fax: 03-5541-5879.

### (Example 1)

Acquirement of candidate cDNA fragments encoding a novel protein having acyltransferase activity expressed in carnation petals

The carnation variety Lucia (pink carnation as a breeding line of Kirin Agribio Co., Ltd., which comprises pelargonidin-3-malyl glucosides, acylated anthocyanins, as main pigments) was cultivated and caused to bloom in a greenhouse according to a standard method. mRNA was prepared from petals by use of RNeasy (Qiagen). Total cDNA was synthesized by use of SuperScript First-Strand System (Invitrogen Corporation).

This cDNA was subjected to PCR (conditions: 95°C for 5 min., 30 cycles of (95°C for 30 sec., 55°C for 30 sec., and 72°C for 1 min.), and 72° for 10 min.) using primers [U592: ATGATTTGGCTTACAGGNGGNCCNGGNTG (SEQ ID NO: 4) and U593: GCTGTRTGNCCNGCNCCYTT (SEQ ID NO: 5)] prepared on the basis of the gene information of a glucose/isobutyric acid transfer protein [Li et al., PNAS, 97, 6902-6907 (2000)] synthesizing tomato acyl acetals (substances resistant to insects), a serine carboxypeptidase [Genbank ACCESSION XP_474646] having homology to the sequence thereof, which is predicted from rice genomic sequence, a barley serine carboxypeptidase [Genbank ACCESSION CAA70816] also having homology to the sequence thereof, and an *Arabidopsis* serine carboxypeptidase SNG2 [Genbank ACCESSION NP_568215] also having homology to the sequence thereof. A PCR enzyme used in this PCR and in subsequent experiments was ExTaq manufactured by Takara Shuzo Co., Ltd. The amplification products were separated by electrophoresis at 100 V for 20 minutes using a 1% agarose gel and visualized by ethidium bromide staining. The DNA fragments were confirmed to be amplified with predicted molecular weights of approximately 1 kb. The obtained amplification products were cloned by use of TOPO TA Cloning Kit for Sequencing (manufactured by Invitrogen Corporation) and sequenced for determining the nucleotide sequences by use of ABI310 (Applied Biosystems). Twenty-four nucleotide sequences of clones were thus determined to obtain 4 types of clones, AT3-1 (13 clones), AT3-2 (9 clones), AT3-3 (1 clone), and AT3-4 (1 clone). Primers specific to each of these four types of clones were synthesized. [AT3-1: U609: GGTTGCTCTGCTTTCTCTGGCCTC (SEQ ID NO: 6) and U610: TTAACTGTAGCATAAACTAAGCGG (SEQ ID NO: 7), AT3-2: U611: GGGTGCTCTTCTTGGAACGGTCTCG (SEQ ID NO: 8) and U612: CCCTTGACTGTCGCGTACGTCAAAG (SEQ ID NO: 9), AT3-3: U622: CTGCTTTTTCTGGTTTAGCC (SEQ ID NO: 10) and U623: CAGTAGTATAGGTTAACCGG (SEQ ID NO: 11), and AT3-4: U624: GCTCTGCTTTGTCGGGCCTC (SEQ ID NO: 12) and U625: ACCGTAGCATAGACTAATCG (SEQ ID NO: 13)]

### (Example 2)

Isolation of a gene encoding a novel protein having acyltransferase activity

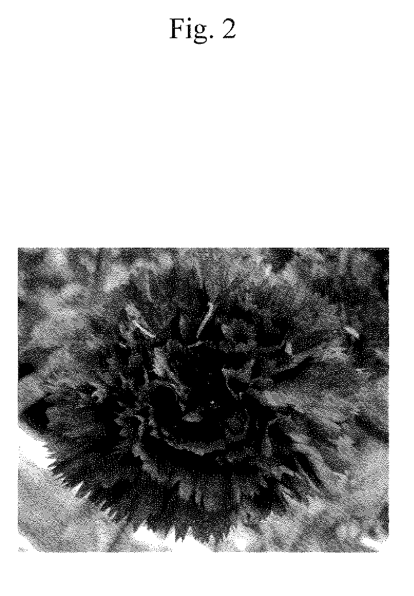

MFA1 and MFA2 lines are breeding lines of Kirin Agribio Co., Ltd. The MFA1 line (FIG. 2), its 4 progeny lines (MFA1-1 to MFA1-4, all from Kirin Agribio Co., Ltd.), the MFA2 line, and its 2 progeny lines (MFA2-1 and MFA2-2, all from Kirin Agribio Co., Ltd.) are revolutionary bicolor lines, all of which have petals where a base color is pale purple and comprises pelargonidin-3,5-diglucosides, non-acylated anthocyanins, as a main pigment, and a variegated spot color is pink and comprises cyclic 5-3-malyl pelargonidins, acylated anthocyanins, as a main pigment. Genomic DNA was extracted from leaves by use of DNeasy (Qiagen). Genomic DNA was also extracted as a control from the leaves of the carnation variety Lucia. The genomic DNAs from the leaves of the MFA1 and MFA2 lines and Lucia were subjected to genome comparison analysis using the primers synthesized in Example 1. The use of the AT3-1-specific primers (SEQ ID NOs: 6 and 7) showed that a specific insertion sequence was observed in the MFA1 line but was not inherited to the 4 progeny lines (MFA1-1 to MFA1-4). The use of the AT3-2- and AT3-4-specific primers (SEQ ID NOs: 8 and 9 and SEQ ID NOs: 12 and 13, respectively) showed that a specific insertion was not observed in the MFA1 and MFA2 lines. The use of the AT3-3-specific primer (SEQ ID NO: 10) showed that an insertion fragment of approximately 4 kb was observed to be present in both of the MFA1 and MFA2 lines. Analysis using the same primer (AT3-3-specific primers (SEQ ID NOs: 10 and 11)) showed that the insertion fragment was inherited to their respective progeny lines (MFA1-1 to MFA1-4 and MFA2-1 and MFA2-2, respectively).

To determine the full-length sequence of the AT3-3 gene, the mRNA obtained from Lucia in Example 1 was sequenced for determining the full-length cDNA sequence (SEQ ID NO: 2) by use of GeneRacer Kit (Invitrogen Corporation). FIG. 3 shows the amino acid homology between a novel acyltransferase protein (SEQ ID NO: 1) encoded by the gene and a glucose/isobutyric acid transfer protein, which synthesizes tomato acyl acetals (substances resistant to insects) and was used to isolate the novel acyltransferase protein-encoding gene. These amino acid sequences exhibit 44.6% homology as a whole, though partial more homology is observed. Most of plant acyltransferases are encoded by a group of plant BAHD acyltransferase genes described in D'Auria, Curr. Opin. Plant Biol., 9, 331-340 (2006). Surprisingly, any homology of the present gene to the group of plant BAHD acyltransferase genes was not observed.

### (Example 3)

MFA1 line- and MFA2 line-specific insertion sequences located in the gene encoding a novel acyltransferase protein

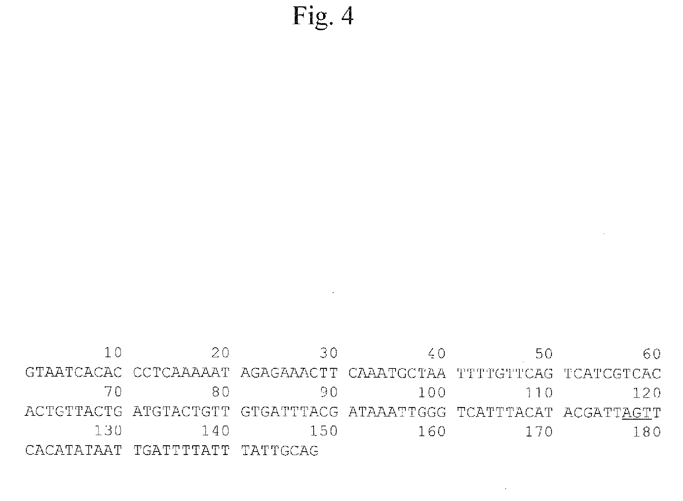

Genomic fragments from the leaves of the MFA1 and MFA2 lines were amplified by use of primers [U627: GAATATGAACGTCGCGTATCAC (SEQ ID NO: 14) and U628: CATTGCAACTGATCTTGGCCG (SEQ ID NO: 15)] prepared on the basis of the full-length cDNA sequence (SEQ ID NO: 2) of Lucia. The amplification products were cloned by use of TOPO TA Cloning Kit for Sequencing and sequenced for determining the nucleotide sequences. The insertion fragment was located within a 148-bp intron (FIG 4) between bases at the positions 732 and 733 of the nucleotide sequence set forth in SEQ ID NO: 2 and caused the duplication of AGT at the positions 117 to 119 of the sequence shown in FIG. 4. Specifically, the insertion fragment of approximately 3.6 kb was inserted therein in the form exhibiting Target site duplication (TSD) of the AGT sequence. The nucleotide sequence of the insertion fragment is shown in SEQ ID NO: 3. The same insertions were observed at the same positions of the MFA1 and MFA2 lines. The affinity between the MFA1 and MFA2 lines in terms of breeding is unknown. However, it was expected that these lines were bred from those having the same insertion fragments.

### (Example 4)

Excision of the specific insertion fragment in a variegated spot portion

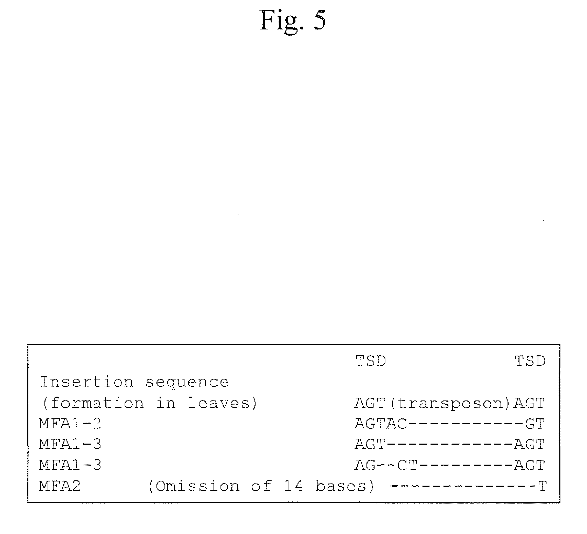

Genomic DNA was extracted from pink variegated spot portions in the petals of two progeny lines (MFA1-2 and MFA1-3) of the MFA1 line and the MFA2 line by use of DNeasy. The Genomic fragments were amplified by use of the primers (U627 and U628) used in Example 3. The amplification products were cloned by use of TOPO TA Cloning Kit for Sequencing and sequenced for determining the nucleotide sequences. As a result, the insertion fragment of Example 3 was excised from all of the petals, and foot prints were observed (FIG. 5).

These results demonstrated that the specific insertion fragment found in Example 3 was transposon. The inserted transposon remains in leaves or in petals accumulating therein non-acylated anthocyanins. Therefore, the gene encoding a novel protein having acyltransferase activity does not function therein, resulting in the accumulation of non-acylated anthocyanins. On the other hand, the transposon has been excised in variegated spots accumulating therein acylated anthocyanins. As a result, it was demonstrated that the gene encoding a protein having acyltransferase activity functions therein, resulting in conversion to acylated anthocyanins.

### Industrial Applicability

The present invention relates to a novel protein having acyltransferase activity, a gene encoding the same, and a method for producing a plant having the gene. Specifically, according to the present invention, it has become possible to breed an unprecedented plant containing acylated anthocyanins comprising malic acids, i.e., a plant having a novel flower color, by introducing the gene encoding a novel protein having acyltransferase activity into a plant not containing such gene.

According to the present invention, a novel protein having acyltransferase activity, the gene encoding the same, and a method of producing a plant having the gene are provided. In addition, the gene encoding the novel protein having acyltransferase activity of the present invention is introduced into a plant lacking such gene or a plant not having acyltransferase activity, such as a plant lacking a novel acyltransferase or a plant in which a novel acyltransferase does not function (e.g., a plant other than a plant belonging to the genus Dianthus (e.g., a carnation)). Thus, breeding of an unprecedented plant containing anthocyanins comprising malic acids as acyl groups (i.e., a plant having a novel flower color) can be realized.

The range of flower colors can be expanded by changing the flower colors of flowering plants by use of the novel protein having acyltransferase activity of the present invention and the method of producing a plant by use of the gene encoding the same. Thus, the protein and the method of the present invention are useful for development of ornamental plants having novel properties.

All publications, patents, and patent applications cited herein are incorporated herein by reference in their entirety.
Free Text of Sequence Listing
SEQ ID NOS: 4 to 15 (primers)

## Claims

1. A protein which is the following (a) or (b):
(a) a protein consisting of the amino acid sequence set forth in SEQ ID NO: 1; or
(b) a protein consisting of an amino acid sequence derived from the amino acid sequence set forth in SEQ ID NO: 1 by deletion, substitution, insertion, or addition of 1 or more amino acid(s) and having the activity of transferring malic acids as acyl groups to anthocyanins.

2. A gene consisting of DNA which is one of the following (c) to (f):
(c) DNA consisting of the nucleotide sequence set forth in SEQ ID NO: 2;
(d) DNA which hybridizes under stringent conditions to DNA consisting of a nucleotide sequence complementary to the nucleotide sequence set forth in SEQ ID NO: 2 and encodes a protein having the activity of transferring malic acids as acyl groups to anthocyanins;
(e) DNA which consists of a nucleotide sequence having a homology of 80% or higher to the nucleotide sequence set forth in SEQ ID NO: 2 and encodes a protein having the activity of transferring malic acids as acyl groups to anthocyanins; or
(f) DNA consisting of a degenerate isomer of the nucleotide sequence set forth in SEQ ID NO: 2.

3. A recombinant vector containing the gene according to claim 2.

4. A plant cell to which the recombinant vector according to claim 3 has been introduced.

5. A method for producing a gene recombinant plant having petals with a novel flower color, consisting of introducing the gene according to claim 2 into a plant cell so as to regenerate a plant from the plant cell.
